# EUROPEAN PATENT SPECIFICATION

(11) **EP 4 313 224 B1**
(45) Date of publication and mention of the grant of the patent: **29.01.2025**
(21) Application number: 22711925.2
(22) Date of filing: 03.03.2022
(51) Int. Cl.: A61M 16/00, A61M 21/02

(54) **VENTILATION APPARATUS**
LÜFTUNGSGERÄT
APPAREIL DE VENTILATION

(30) Priority: 02.04.2021 US 202163170202 P
(43) Date of publication of application: 07.02.2024
(73) Proprietor: Koninklijke Philips N.V., 5656 AG Eindhoven (NL)
(72) Inventor: DELLIMORE, Kiran, Hamilton, J., 5656 AG Eindhoven (NL); SAINI, Privender, Kaur, 5656 AG Eindhoven (NL); FONSECA, Pedro, Miguel, Ferreira dos Santos da, 5656 AG Eindhoven (NL); GRASSI, Angela, 5656 AG Eindhoven (NL); TROXELL, David, Aaron, 5656 AG Eindhoven (NL); PRIORI, Rita, 5656 AG Eindhoven (NL)
(74) Representative: Philips Intellectual Property & Standards
(86) International application number: PCT/EP2022/055390
(87) International publication number: WO 2022/207229

(56) References cited:
- EP-B1- 1 893 264
- WO-A1-2019/157563
- WO-A1-2022/058967
- US-A1- 2009 038 616
- US-A1- 2021 046 267

## Description

### FIELD OF THE INVENTION

This invention relates to ventilation therapy, and particular relates to improving user compliance by improving habituation to the therapy.

### BACKGROUND OF THE INVENTION

Patients who are new to continuous positive airway pressure (CPAP) and non-invasive ventilation (NIV) therapy often take several months to habituate to the therapy due to discomfort caused by the patient interface (i.e., mask), difficulty in adjusting to the sensations of the therapy itself, as well as other factors which make it hard for them to achieve and maintain sleep at night. Examples of the latter include loud operation of the ventilation device and noise produced by high flow air leaks due to poor fit of the patient interface.

Moreover, other significant impediments to patient comfort and compliance include the treatment obstructing the ability of the patient to communicate with care givers, and disturbance of the patient's bed partner. This leads many new patients to reject the therapy early on and to be less compliant with their mask therapy at least in the beginning, which may also have adverse implications for long-term therapy compliance.

Currently, most new NIV patients do not receive sufficient support, education or training at the initiation of therapy to ensure appropriate acceptance and habituation. In many instances, patients are introduced to the new therapy in a hospital setting without much explanation or during a single session at home with a respiratory technologist in which the patient interface is fitted (at relatively low pressure and high flow) and the ventilation device settings are optimized. After this, they are on their own and are for example recommended to use it at least 5-6 hours per night while sleeping.

The American Academy of Sleep Medicine clinical guidelines indicate that all potential positive airway pressure (PAP) titration candidates should receive adequate PAP education, hands-on demonstration, careful mask fitting, and acclimatization prior to titration. PAP desensitization, generally conducted during daytime sessions with a sleep technologist, is used when patients who would benefit from PAP therapy have difficulty acclimating to therapy.

PAP desensitization is generally undertaken with patients who were unable to tolerate PAP during a titration study or they cannot use the PAP at home due to claustrophobia or inability to tolerate PAP pressures. PAP desensitization can be performed in a sleep clinic, sleep center, or the patient's home. It is most often initiated in the sleep clinic or sleep center, and continued in the patient's home until the patient has acclimated to the mask and pressure sufficiently to return for a PAP titration study. Because the patient is awake and aware, the desensitization process can be difficult.

With regard to compliance, for example to a CPAP therapy, most insurance guidelines require, for reimbursement purposes, that the patient is compliant with the therapy in a period of 90 days; compliance is established at a minimum of 4 hours per night, for at least 21 nights during a consecutive 30-day period during the 90-day trial period. An estimated 25% of the patients are not compliant during the first 90 days, and drop-out increases to 30-50% during the first year.

There is therefore a need to habituate new patients to the mask therapy to increase compliance and successful habituation in the short and long term.

EP 1893264 B1 describes a method of acclimatizing a user to continuous positive airway pressure (CPAP) therapy. The method comprises: determining a full therapeutic pressure, and applying a sub-therapeutic pressure for the duration of a first session.

US 2009/038616 A1 describes systems for acclimatizing a user to positive airway pressure (PAP) therapy. Generally, a sub-therapeutic treatment pressure is provided initially.

WO 2019/157563 A1 describes a method for treating hyperarousal disorder, comprising determining settings that control respiratory therapy for slowing a patient's breathing.

US 2021/046267 A1 describes methods providing automated controls for a respiratory pressure therapy device, such as a servo-ventilator. For example, a controller of a respiratory pressure therapy device may control application of pressure support ventilation therapy to an airway of a patient.

### SUMMARY OF THE INVENTION

The invention is defined by the claims.

According to examples in accordance with an aspect of the invention, there is provided a ventilation apparatus for providing breathing therapy, comprising:
a breathing gas delivery system for delivering breathing gas to a user of the apparatus, with a controllable pressure and flow;
a pressure sensor for monitoring a breathing pressure;
a flow sensor for monitoring a breathing flow; and
a processor adapted to:
   process the monitored breathing pressure and the monitored breathing flow to derive a respiration parameter which is indicative of a relaxation state of the user;
   estimate a state of relaxation of the user during delivery of breathing therapy based on the respiration parameter; and
   configure settings of the ventilation apparatus in dependence on the estimated state of relaxation for assisting the user in habituating to the breathing therapy;
wherein the processor is adapted to configure said settings of the ventilation apparatus to control at least an expiratory pressure level;
wherein the processor is adapted to configure the ventilation apparatus settings such as to vary the settings incrementally in steps from a sub-therapeutic level to a therapeutic level,
wherein the processor is adapted to so vary the settings incrementally to increase the pressure level from a sub-therapeutic to therapeutic level only while the user remains above a defined relaxation state threshold thₘᵢₙ;
wherein, responsive to the relaxation state falling below the relaxation state threshold thₘᵢₙ, the processor is adapted to lower the ventilation pressure in order to allow the user to return to a more relaxed state.

In this way, the system adapts ventilator settings based on the user's relaxation level. The aim is to habituate the user to the therapy in the most effective way, by using their relaxation level as a feedback parameter for controlling at least the ventilation settings.

The feedback control thus implements behavioral conditioning, for providing graded exposure and systematic desensitization for the purposes of habituating patients to tolerate mask therapy.

The processor may be adapted to set a minimum relaxation level, and adapt the settings of the ventilation apparatus in response to the relaxation level dropping below the minimum level. Thus, when the user is agitated or stressed, the ventilation settings are lowered so that user is not exposed to stressful settings for prolonged periods. This provides the graded exposure functionality.

The respiration parameter for example may comprise:
a respiration variability (RV); and/or
a heart rate variability (HRV);
a rapid shallow breathing index signal.

These are all surrogate measures of patient relaxation or stress. They may be used in any combination, e.g., HRV and RV

The processor may be adapted to configure the settings to switch between sub-therapeutic and therapeutic levels. The sub-therapeutic levels are only for the habituation process and would not be used during a treatment therapy. They implement the graded exposure approach. A sub-therapeutic setting is for one with a pressure (e.g. expiratory pressure) below a threshold, or a flow below a threshold, or with a defined combination of pressure and flow which combination is below a threshold. A pressure threshold is for example 4 cmH₂O and a flow threshold is for example LPM (liters per minute).

Note that 1 cmH₂O = 98 Pa. Hence, as used in this document, 4 cmH₂O = 392 Pa, and 10 cmH₂O = 981 Pa.

The processor may be adapted to further configure the settings to control one or more of:
the expiratory pressure level;
the flow rate;
the inspiratory rise time; and
the duration of the ventilation device pressure and/or flow periods.

The expiratory pressure is particularly important in the COPD patient population that has significant expiratory flow limitation and/or inspiratory positive airway pressure. However, each of these settings may alter the burden of the ventilation on the user and may thus be used as the controlled feedback parameter to implement the habituation.

The processor may be further adapted to provide audio and/or visual feedback to the user indicating a level of therapy habituation. This enables the user to view their progress.

The apparatus may further comprise additional sensors for providing signals for use in estimating the state of relaxation of the user. Thus, the standard ventilation parameters (flow and pressure) may be supplemented with other parameters.

The additional sensors for example comprise one or more of:
a respiratory effort sensor;
a photoplethysmogram sensor for determining heart rate variability;
a galvanic skin response sensor;
a sweat or saliva sensor for measuring a cortisol level;
a body temperature sensor;
an electromyography (EMG) sensor;
a respiratory effort sensor.

Some of these sensors may be worn at the wrist of the patient and may further confirm the relaxation state of the patient. They may also be used to determine if the intervention measures to promote relaxation are effective.

The apparatus may further comprise an output stimulation device for influencing the relaxation state of the user. This provides ways to relax the patient in addition to the control of the ventilator settings.

The output stimulation device, for example, comprises an audio and/or visual output device for providing one or more of:
red light stimulation;
breathing exercise guidance;
guided meditation;
mask readjustment or change advice;
auditory delivered Eye Movement Desensitization and Reprocessing (EMDR).

Mask readjustment may be suggested based on detected leakage, or other masks may be appropriate for better comfort. Leakage at the patient interface is thereby prevented from disrupting user therapy acclimatization. Biofeedback may be thus used to control user relaxation to enhance therapy acclimatization.

The processor may be further adapted to provide a temperature and/or humidity control signal for controlling the environmental temperature and/or humidity. This provides a further way to assist the relaxation of the user.

The processor is preferably adapted to configure the settings during a pre-therapy training period. Thus, the invention is preferably applied in advance of therapy, thereby to reduce the drop-out rate of the actual, subsequent, therapy.

The invention also provides a computer program comprising computer program code according to claim 11.

### BRIEF DESCRIPTION OF THE DRAWINGS

For a better understanding of the invention, and to show more clearly how it may be carried into effect, reference will now be made, by way of example only, to the accompanying drawings, in which:
Figure 1 shows how a graded exposure to mask settings may be applied;
Figure 2 shows a ventilation apparatus for providing breathing therapy;
Figure 3 shows an example of an estimate of the relaxation level versus time as well as the ventilator device settings for a first control approach;
Figure 4 shows an example of an estimate of the relaxation level versus time as well as the ventilator device settings for a second control approach;
Figure 5 shows an example of an estimate of the relaxation level versus time as well as the ventilator device settings for a third control approach; and
Figure 6 shows an example of an estimate of the relaxation level versus time as well as the ventilator device settings for a fourth control approach.

### DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

The invention provides a ventilation apparatus which has a breathing gas delivery system for delivering breathing gas to a user of the apparatus (i.e. a patient who requires ventilation therapy), with a controllable pressure and flow. The breathing pressure and flow are monitored to derive a respiration variability. A state of relaxation of the user is derived during provision of breathing assistance based on at least the respiration variability. Settings of the ventilation apparatus are then adapted dependence on the estimated state of relaxation for assisting the user in habituating to the breathing assistance, and hence habituating to breathing therapy.

The invention is based on a behavioral conditioning approach, which may use graded exposure and systematic desensitization for the purposes of habituating patients to tolerate mask therapy. Mask therapy, especially in the beginning, is perceived as highly uncomfortable and raises anxiety in patients. To avoid the uncomfortable therapy and accompanying anxiety, patients display evasive behavior: low compliance or even drop-out. Avoiding an anxiety provoking situation is part of human nature; avoidance removes short term distress. However, when a situation is left in order to reduce distress, coming back to the same situation will evoke the same level of distress and can lead to a pattern of fear that, in the context of mask therapy, goes from early low compliance to early drop-out.

A graded exposure and systematic desensitization may be used to help patients adapt more smoothly to mask therapy, thereby preventing low or non-compliance. Graded exposure is an evidence based therapy that is often used in the treatment of phobias. However, it is used in other contexts that may be anxiety or fear provoking. For example, in recent publications, desensitization therapy and graded exposure have been proven useful in pediatric neuroimaging, for the treatment of lower back pain as well in dental practice for dealing with anxieties around dental procedures.

Habituation therapy can thus be applied to many fear or anxiety provoking situations or objects. Graded exposure works by gradually increasing exposure to the anxiety or distress provoking situation. When exposed to a very mild form of the situation or object that gives anxiety, people are able to tolerate this and eventually over time to have no anxiety. The human biological system does not sustain high arousal levels for extensive periods of time, and responds by adapting, and hence bringing down the arousal level given the same stimulus. This process is called habituation. The habituation process can be facilitated or accelerated by active relaxation (systematic desensitization). Note that this is different to simple distraction.

There are four elements that need to be considered in graded exposure. The exposure needs to be:
(i) graded (i.e. with a gradual increase);
(ii) prolonged (i.e. long enough to habituate, which can be anything between 5 and 120 minutes);
(iii) with the absence of distraction (there needs to be a full focus on the anxiety and distress. If a person is relieving distress by other means, like listening to music, habituation cannot occur); and
(iv) repetitive (exposure to the same level needs to happen a few times per week until there is minimal or zero distress).

The invention is based on the application of these concepts to provide habituation to ventilation therapy, i.e. mask-based breathing assistance. This approach can be illustrated via an example of a basic protocol for self-directed graded exposure to mask settings.

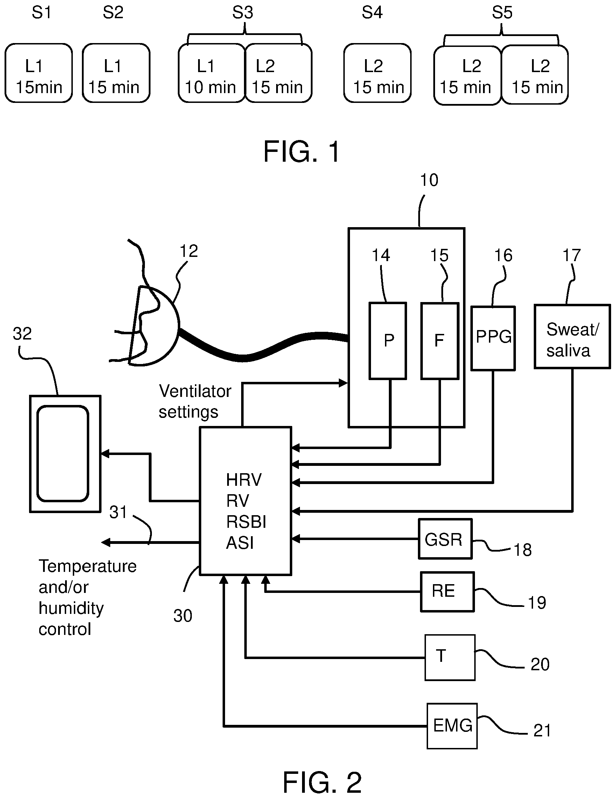

Figure 1 shows how a graded exposure to mask settings may be applied. It shows a sequence of sessions over a five-day period, with one session each day.

Sessions S1 and S2 (on days 1 and 2) make use of a first setting of the ventilator apparatus (level 1), which may be below a therapeutic threshold. This level is determined by the patient as comfortable and exhibiting very low anxiety. This setting is maintained for at least 15 minutes, or until the patient is in an absolute relaxed state.

In session S3 on day 3, the patient starts with level 1 setting and follows through for 10 minutes. If at this point, habituation has set in (i.e. the patient is relaxed), the patient increases the setting to a noticeably higher level (patient-determined), closer to therapeutic, but while still being able to stay calm. In other words, anxiety can go up, but to a manageable level. This level 2 setting is maintained for at least 15 minutes, or however long it takes the patient to adapt (relax).

In session S4 on day 4 the patient starts and ends with level 2, for at least 15 minutes, or again however long it takes to feel relaxed.

In session S5 on day 5 and for subsequent days, the patient follows the above incremental steps and only continues to a next step when he or she finishes two consecutive sessions of the same level in a relaxed state. Habituation may occur more slowly as settings increase and approach therapeutic levels.

The time periods are also increased over time, for example in parallel with the level settings being increased. The exposure is concluded when the patient can tolerate therapeutic settings for at least 30 minutes.

Figure 2 shows a ventilation apparatus for providing breathing therapy which enables an automation of the process described above. The apparatus comprises a breathing gas delivery system 10 for delivering breathing gas to a user of the apparatus (i.e. a patient) via a patient interface, in particular a mask 12.

A pressure sensor 14 monitors a breathing pressure and a flow sensor 15 monitors a breathing flow rate. In manual operation of a ventilator, a clinician can typically either control the flow or pressure delivered to the patient with the other being set by the ventilator. In an automated control approach, both the flow and pressure are controllable by the ventilator. Thus, in this case, the ventilator has a controllable pressure and flow and both are monitored internally by the ventilator as part of the control of the ventilator. Thus, the pressure sensor and flow sensor may be internal parts of the ventilator or external sensors.

A processor 30 processes at least the breathing pressure and breathing flow to derive one or more parameters relating to a relaxation state, such as a respiration variability RV or heart rate variability HRV.

The processor estimates a state of relaxation of the patient during delivery of breathing therapy based on these one or more parameters. Ventilator settings are then configured in dependence on the estimated state of relaxation for assisting the user in habituating to the breathing therapy.

During this estimation, the patient is typically awake or in a light sleep state, and their breathing conditions are monitored to determine their relaxation/stress/anxiety level. The relaxation state is dependent on the autonomic balance between sympathetic and parasympathetic dominance.

This balance is known to be related to the level of relaxation/stress, especially during wakefulness: the more stressed a subject is, the higher his/her sympathetic tone; on the other hand, as a subject progressively relaxes, there is an increase in the parasympathetic activity and hence tone, and corresponding decrease in sympathetic activity.

These tones can be monitored by using known measures of heart rate variability, or when using respiratory signals, by analyzing the patterns of breathing, e.g. captured with a respiratory sensor such as airflow sensor, pressure sensor, thermistors, etc. When monitoring during positive airway pressure treatment or non-invasive ventilation treatment, pressure-based airflow measures may be used.

The autonomic characteristics mentioned above can generally be determined in a number of ways:
i) With any of a known set of heart rate variability features that have been extensively described in relation to autonomic balance;
ii) If respiratory signals are used, with a set of manually engineered features, expressing one or more of the following aspects: breathing rate, regularity of breathing rate, minute ventilation, depth/volume of breathing, timing between inspiration/expiration, length of inspiration vs. expiration, etc. These are all known to change with varying autonomic balance, and can therefore be measured to ascertain whether there is a tendency towards sympathetic or parasympathetic activity.
iii) Using a neural network-based algorithm to automatically determine, from raw cardiac and/or respiratory signals, which features are representative of an autonomic state. Such a neural network can be trained with example signals where the autonomic state is known, e.g. via a protocolized data acquisition where those states are elicited.

Additional sensors may be used to better determine and/or confirm the state of relaxation of the user, to supplement parameters which may be derived from the flow and pressure. Examples shown in Figure 1 are a PPG sensor 16 from which heart rate variability, HRV, may be determined, a sweat or saliva sensor 17 which allows cortisol levels in sweat to be measured, and a galvanic skin response, GSR, sensor 18. A respiratory effort, RE, sensor 19 provides an additional way of sensing breathing characteristics. Figure 1 also shows a body temperature sensor 20, and an electromyography, EMG, sensor 21.

Some different possible combinations of sensor inputs are described below.

Figure 2 also shows an output device 32 in the form of a mobile phone, for providing a display and audio output. This may be used to provide stimulation to the user for improving relaxation such as red light stimulation, breathing exercise guidance, guided meditation, or other advice such as mask readjustment or mask change advice. It may also provide auditory delivered Eye Movement Desensitization and Reprocessing (EMDR). There may also be a tactile output, which may also provide EMDR. An additional (optional) output from the processor 30 is a signal 31 for controlling an external climate control system, in particular for controlling the temperature and/or humidity of the environment in which the patient is being treated.

A first example of the operation of the apparatus will be explained in more detail, based on determining user therapy acclimatization by measuring a relaxation level based on respiration variability, RV.

The level of habituation of a new user to a non-invasive ventilation, NIV, therapy or continuous positive airway pressure, CPAP, therapy is estimated in this first example by inferring the user's relaxation state from their respiration variability, RV, derived from processing the respiration signals acquired from the ventilation apparatus during a simulated therapy. This simulated therapy may be considered to be a therapy training. The therapy training takes place when a user is in an awake or light sleep state (e.g., a siesta or nap), undergoing simulated therapy. In the simulated therapy, the patient is wearing a NIV or CPAP mask, but the ventilation settings may be sub-therapeutic, as explained above.

Thus, the pressure levels and flow levels may be below respective thresholds which may be considered to define the lower limits for a therapy.

The simulated therapy may be applied for progressively longer periods of time, e.g., 15, 30, 45, 60 minutes or longer (although of course other time increments such 5, 10, 20 or 30 minutes may of course be used). After habituation, gradually higher settings may be applied, reaching therapeutic levels. During therapy training, the user's relaxation state is monitored continuously (or semi-continuously, i.e. periodically) by processing the respiration signals to determine the user's RV. Based on the user's relaxation level, the ventilation device settings during the therapy training may be adapted in several different ways, based on level settings (pressure and flow) and timings.

In addition, audio-visual feedback may be provided to the user related to their level of therapy habituation. For example, a green light on the ventilation device may indicate the user is in a relaxed state. A key assumption is that users who are better able to control their relaxation (i.e., better control their response to distress) will have higher therapy tolerance and a more predictable habituation timeline, while users who are less able to control their relaxation (i.e., worse control of their response to distress) will have a lower initial therapy tolerance and a longer habituation timeline.

This approach is underpinned by psychological literature on exposure therapy and systematic desensitization as described above.

This first example makes use of the assessment of respiration variability, determined from the respiration signals. RV describes a family of physiological characteristics, or features, measured from signals related to respiration, such as measures of oral, nasal or oro-nasal airflow. For this purpose, the pressure sensor 14 may be integrated in the ventilator device as shown, or instead in the mask 12.

Alternatively, the respiratory effort sensor 19 may be used. There are various options for respiratory effort sensing, such as:
a belt worn around the thorax with a microphone positioned on the suprasternal notch;
intra-costal surface EMG sensing;
an accelerometer or gyroscope, or other acceleration, displacement or movement sensor mounted on, or close to the thorax (e.g. on the bed, under the mattress, etc.). A gyroscope may be used to measure rotations associated with breathing, and these provide adequate surrogate measures of respiratory effort.

Each of these sensor approaches may be used to measure a specific aspect of the breathing of the subject or other physiological signals, such as variations in tidal volume, variations in peak inspiratory and expiratory amplitude, the duration of inspirations and expirations and ratios between these, changes in the shape of the breathing amplitudes, etc. The monitored features are traditionally calculated either over windows of a given length (a few seconds to a few minutes), or in windows of variable length, defined by a determined number of breaths.

The features are computed based on either the actual respiratory signal (e.g. airflow), or on a time series comprising breath-to-breath characteristics.

Respiratory variability can then be expressed by any combination of sample statistics (e.g. average, percentiles, range, standard deviation, etc.), or by the result of time-frequency analysis (e.g. using short-time Fourier transform, or discrete or continuous wavelet transforms). These features describe how regular (or irregular) breathing is throughout the measurement period and also some characteristic values (e.g. mean breathing rate, mean tidal volume) during that period.

Alternatively, as mentioned above, instead of (or in addition to) manually engineered features, neural networks may be used that learn, as part of their training procedure, which features better express and/or quantify, from the raw respiratory signal, the autonomic balance (and stress-relaxation balance) of a given subject at a given time.

The aim of the monitoring is to personalize and smoothen therapy habituation for new NIV and CPAP users. This is achieved based on the assumption that the rate of therapy acclimatization is linked to the user's individual relaxation levels during therapy training. As relaxation is the indicator of habituation, the therapy tolerance can be increased with more confidence ensuring most efficient graded exposure and habituation. This ensures smoother therapy habituation and better compliance during the initial period when the user is first introduced to the therapy.

This in turn leads to reduced therapy resistance and aversion (i.e., potential drop-out).

To accomplish this personalization, a personalized minimum relaxation threshold, thₘᵢₙ, may be defined and utilized as a cut-off when challenging the user's therapy tolerance. This threshold can be determined in one of several ways. For example, it may be based on:
i) an initial learning phase in which the user is asked to wear the mask at a low sub-therapeutic setting, until they feel uncomfortable. Their level of relaxation is measured based on the RV during this time and thₘᵢₙ is set as the relaxation level just before the user removes the mask.
ii) a population average of relaxation thresholds from a large group of users with similar characteristics such as age, gender, disease severity, etc.
iii) a one-time user feedback test in which the user is asked to provide feedback on their level of relaxation during simulated therapy in which settings are varied stepwise from sub-therapeutic to therapeutic levels. The user's measured level of relaxation based on the RV is then correlated to their self-reported level of relaxation to determine thₘᵢₙ by finding the minimum measured relaxation level at which the user reports feeling relaxed during simulated therapy.

Preferably the obtained threshold thₘᵢₙ corresponds to a level of relaxation that when crossed is barely noticeable to the patient, i.e., still within a comfort zone of the individual patient. It should not result in a sudden or sharp decrease in the relaxation level of the patient (or conversely a sharp rise in their anxiety or distress). Once obtained, thₘᵢₙ, is used as a cut-off to determine whether to maintain or adapt simulated therapy.

If the user's estimated relaxation remains consistently above thₘᵢₙ (i.e., after repeated exposure through therapy training), then simulated therapy settings may be maintained or adapted to be closer to the therapeutic levels and/or the duration of therapy training may be extended. If the user's relaxation falls below thₘᵢₙ, then simulated therapy settings may be gradually adapted to be further from therapeutic levels (i.e., stepped down) and/or the duration of therapy training may be shortened.

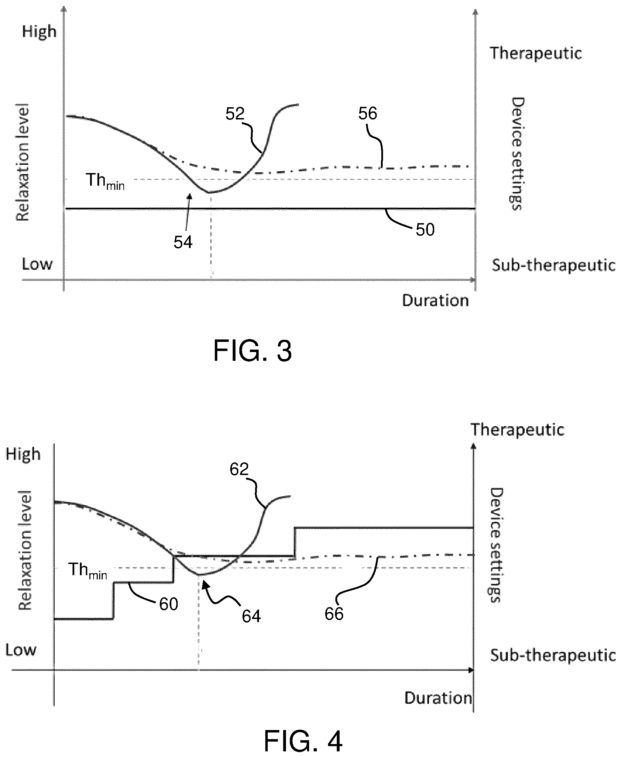

To illustrate the functioning of the apparatus, exemplary scenarios are described with reference to Figures 3 to 5.

In a simplest case, a situation is considered in which therapy training is initially performed with the user wearing the mask, with the ventilation device at a fixed, low, sub-therapeutic setting (such as at a pressure of 4 cmH₂O and a flow rate of 20 LPM); for progressively longer periods of time up to two hours, provided the user's relaxation level remains above a set minimum threshold.

Figure 3 shows an estimate of the relaxation level (left y-axis, arbitrary units) versus time (x-axis) for two subjects. The right y-axis shows a general level of the device settings, indicating if the device settings are therapeutic or sub-therapeutic.

Plot 50 shows the device settings, as constant over time. Plot 52 shows a user with a low therapy habituation. Their relaxation level may after some time fall below thₘᵢₙ, as shown at point 54. This leads therapy training intensity to be diminished, or the duration to be curtailed, e.g., via application of a short duration wind-down strategy.

In contrast, plot 56 shows a user with a higher level of habituation. Their relaxation remains for longer a time above thₘᵢₙ thereby allowing a longer period of therapy training to be realized. Each therapy training session should be preferably ended at the highest possible relaxation state.

Figure 4 shows another example in which therapy training is performed with the user wearing the mask and the ventilator settings are varied incrementally in steps from sub-therapeutic to therapeutic, such as from a pressure of 4 cmH₂O to 10 cmH₂O, and a flow rate from 20 LPM to 30 LPM, over a three-hour period. This is represented by the device settings plot 60. In this case, the ventilation device settings are adjusted incrementally to increase the pressure and the flow rate from sub-therapeutic to therapeutic levels only if the user remains above the relaxation threshold thₘᵢₙ as is the case for plot 66 for a user with high habituation. This allows the device settings to reach a closer therapeutic level and therapy training to last longer.

If the user has lower therapy habituation as shown in plot 62 then their relaxation level may again after some time fall below thₘᵢₙ, which may again lead the therapy training to be curtailed at point 64 after only two incremental changes in the device settings.

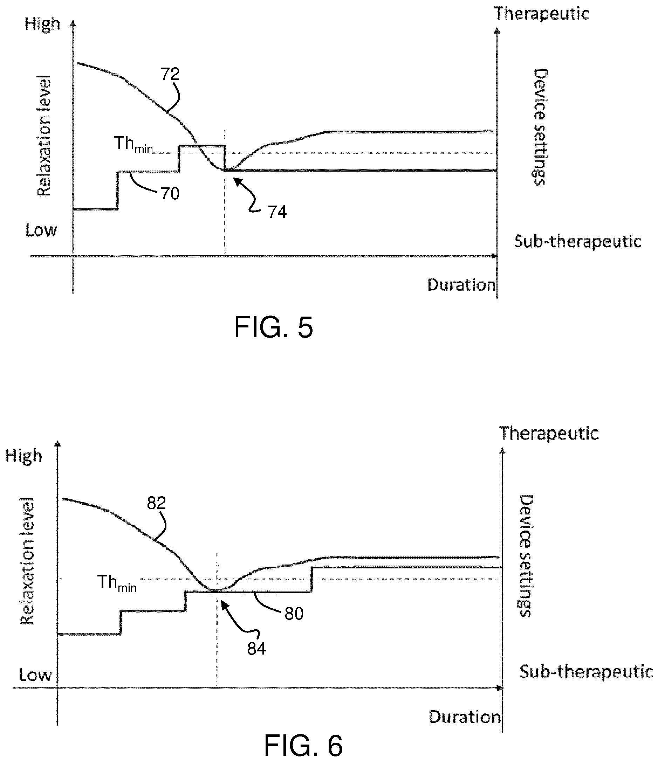

Figure 5 shows an example in which a user is undergoing NIV therapy training while the ventilator settings are varied incrementally in steps from sub-therapeutic to therapeutic, such as from a pressure of 4 cmHzO towards 10 cmH₂O, and a flow rate from 20 LPM towards 30 LPM, over a specified one to two-hour period. The ventilator settings are shown as plot 70. Alternatively, the pressure and flow rate may be kept constant while the inspiratory rise time is adjusted (e.g., making it shorter or longer).

However, in this example, the relaxation level of the user, shown as plot 72, does not remain above the minimum threshold thₘᵢₙ as the settings are incrementally changed. However, instead of terminating therapy after thₘᵢₙ is breached, the system lowers the ventilation pressure and flow rate at step 74 in order to allow the patient to return to a more relaxed state.

The example above is based on estimating a relaxation level based on respiration variation.

Another example is to determine the user's relaxation level additionally or alternatively from one or more of heart rate variability (HRV), galvanic skin response (GSR), body temperature or electromyogram (EMG) signals obtained from a wirelessly connected device worn by the user, e.g., a smart watch, wristband or patch.

HRV, GSR, body temperature and EMG are well-known measures of stress and relaxation, which can be used to confirm the relaxation level determined from the RV, or to augment the RV determined relaxation level in situations in which the RV relaxation measure is less reliable or unavailable.

In particular, GSR, HRV body temperature, and EMG may also be useful for the initial stages of therapy training in which the user wears the NIV or CPAP mask with the ventilation switched off, which means that the measure of RV is unavailable, in particular if respiration cannot be obtained with other, additional sensors (e.g. respiratory belts worn around the thorax/abdomen, acceleration, displacement or movement sensors mounted on or close to the chest, etc.), or as a surrogate measurement from e.g. the amplitude modulations of a PPG signal.

The most reliable relaxation measure at a given moment during therapy training may be selected if two or more measures of user relaxation are available from different sensor signals that are available.

An alternative approach to the use of RV (and optionally also HRV) is to use the rapid shallow breathing index, RSBI, signal as a surrogate measure for the stress experienced by the patient while undergoing the mask-based therapy.

RSBI is an index which is widely used in the weaning of patients on mechanical ventilation, in high acuity settings such as the intensive care unit. It is defined as the ratio of respiratory frequency to tidal volume (f/TV). Patients on a ventilator who cannot tolerate independent breathing tend to breathe rapidly (high frequency) and shallowly (low tidal volume), and will therefore have a high RSBI. The higher the RSBI, the more stressed or distressed the patient is generally considered to be. Hence, by adapting the mask-therapy settings according to changes in the patient's RSBI it is possible to guide their therapy habituation, in a similar manner as described previously for RV and HRV.

As an example, a 70 kg patient may be considered who is undergoing therapy training with as NIV mask setting at level 2. If they have a respiratory rate of 25 BPM and an average tidal volume of 250 mL/breath, this will imply a value of RSBI = (25 BPM)/(0.25 L) = 100 BPM/L. This is a high value of RSBI and thus would indicate that the patient is stressed and not relaxed at this setting. The system would then lower the therapy settings, for example to level 1. At that level, the patient is able to breath at 12 BPM, with a tidal volume of 420 mL/breath, resulting in an RSBI = (12 BPM)/(0.420 L) = 28 BPM/L. This is a much lower of RSBI which indicates that the patient is in a less stressed/more relaxed state, suitable for therapy habituation.

Conversely, the increased RSBI index may indicate a heightened anxiety state due to a perception of increased dyspnea and air hunger. In this instance the system may increase the therapy settings (pressure/flow) to satisfy patient demand resulting in a decreased frequency, an increased tidal volume, and a lower RSBI confirming that the patient moved to a more relaxed state.

Another surrogate measure for the stress/distress experienced by the patient while undergoing NIV therapy in particular (which is typically delivered in a pressure support ventilation or pressure support ventilation mode, where the patient can trigger the ventilator), is patient-ventilator asynchrony. The degree of synchrony between the patient and ventilator action is an important aspect that can impair patient comfort and hence the ability to adapt to ventilation. Asynchronies occur when patients are "fighting with the ventilator", as a result of a mismatch between the patient's respiratory efforts and the ventilator-delivered breaths. Several different types of asynchronies can arise, e.g., due to ineffective triggering, auto-triggering, trigger delay, expiratory muscle contraction, etc.

In the intensive care unit, asynchronies during mechanical ventilation have been shown to be associated with higher mortality, and have been linked with different adverse outcomes in the literature as well as with anxiety. Asynchronies events can be detected and calculated for instance with the asynchrony index, which is defined as the number of asynchrony events divided by the total breathing frequency. The total breathing frequency is computed as the sum of the number of ventilator cycles, triggered or not, and of wasted efforts, as proposed by Thille et al., "Patient-ventilator asynchrony during assisted mechanical ventilation", Intensive Care Med. 2006;32(10): 1515-1522.

The asynchrony index may be used as a surrogate measure of discomfort during mechanical ventilation. The more a person gets used to interacting with the ventilator, the lower the asynchrony index will be and vice versa. An asynchrony index of >10% is considered high and indicative of patient stress/distress, while a value ≤2% is considered low and corresponds to the patient being in a more relaxed state.

Figure 2 also shows a sweat sensor 17. This may be used to determine the level of cortisol within sweat or and saliva. This provides a measure of the stress experienced while using the mask-based therapy device. This may be considered to be an inverse to a level of relaxation, and this is also indicative of a relaxation state.

Focusing on the measurement of the stress provides a more direct access to the habituation process. Given the correlation between cortisol and stress responses, cortisol levels may be measured before, during and after the graded exposure sessions. While there are many possible ways to do this, in a home setting the most burden-free and unobtrusive way to achieve this in a semi-continuous manner is via sampling of sweat or saliva. This can be accomplished by integrating a sweat sensor (i.e., electrochemical sensor) in the mouth piece (in the case of saliva sampling) or in the mask itself (in the case of sweat sampling).

Cortisol is routinely and reliably measured in saliva including in small quantities, while measurement in sweat has been shown to strongly correlate with blood and saliva cortisol levels. When the cortisol level measured in sweat or saliva starts to decline during the therapy training session relative to pre-session levels (e.g., measured at the start of the session when the patient first puts on the mask and before ventilation is activated), this is an indication to intensify the exposure. Conversely, if the cortisol level rises steadily and remains high relative to the pre-session level then this is an indication to lower the exposure.

The therapy acclimatization of the user may also be improved during therapy training by taking other measures which aim to ensure that the user always remains at a high relaxation level above the minimum threshold. This may be accomplished by applying various audio-visual and olfactory relaxation techniques (e.g., using the device 32) throughout the therapy training or at specific moments during the therapy training to increase the user's relaxation level as their therapy tolerance is being gradually challenged.

Figure 6 shows an example in which a user is undergoing NIV therapy training while the ventilator settings are varied incrementally in steps from sub-therapeutic to therapeutic, such as from a pressure of 4 cmH₂O towards 10 cmH₂O, and a flow rate from 20 LPM towards 30 LPM, over a specified one to two-hour period. The ventilator settings are shown as plot 80. The relaxation level is shown as plot 82.

When the relaxation level drops below the threshold thₘᵢₙ, relaxation strategies are applied, at point 84. These relaxation strategies may include but are not limited to red light stimulation, guiding the user to perform breathing exercises, applying visual relaxers, environmental temperature and/or humidity adjustment (e.g., cooling with a fan or lowering of a thermostat setting), application of tactile or auditory delivered Eye Movement Desensitization and Reprocessing (EMDR), etc.

These audio-visual strategies may be further combined to advantage with olfactory stimuli, such as incense, smelling salts, aromatic candles, etc. In this case, a trigger or prompt may be sent to the user to recommend them to utilize a preferred olfactory method during therapy training.

In another example, therapy acclimatization of the user during therapy training is enhanced by ensuring that the user receives biofeedback of their relaxation state. In particular, the device 32 may be used to provide a visual display, or other visual means may be used such as ambient light, to display how relaxed a patient is, and which factors contribute to that relaxation level.

As the patient is moved to a next level of habituation, the user is encouraged to anticipate and proactively 'relax' to stay within a safe relaxation-range before moving to a higher flow or intensity setting.

Alternatively, a purer form of habituation may be adopted by which the patient focuses on the anxiety and the stress and allow the user to control anxiety and distress with more `in the moment' techniques like mindfulness. This means the user is focused on the stimulus and thinks about what they are really experiencing, and how this is affecting them. In addition, the user may receive biofeedback of the state of anxiety or arousal. In particular, we propose a visual display or other visual means like ambient light may be used to display how anxious a patient is and lets the user see how stress levels are going down as time progresses. When the patient has reached an acceptable level of anxiety, the user is encouraged to increase the therapy settings.

The light or biofeedback mechanism in this example is used to visualize and guide the user's level of relaxation (or alternatively anxiety distress) as opposed to being used as a stimulus to induce relaxation.

Air leakage at the patient interface may also be a cause of disrupting user therapy acclimatization. If leakage occurs at the patient interface, the ventilator will typically automatically compensate by blowing harder to increase the pressure and thereby improve the sealing at the patient interface. However, this leakage compensation will likely adversely affect patient comfort and prevent the user from entering into a relaxed state leading to poor habituation during therapy training. This situation may be prevented by adopting a preliminary step prior to beginning pressure habituation, in which a check is performed for leakage compensation at the patient interface. If a high level of compensation is required (e.g., >20% pressure compensation) then feedback will be given to the user that the mask is poorly fitting and should be adjusted prior to performing further therapy training.

This invention can be applied in domestic settings to help acclimatize patients who are new to CPAP or NIV therapy. It is also useful in any application in which a user must become habituated to a mask-based therapy.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality.

The mere fact that certain measures are recited in mutually different dependent claims does not indicate that a combination of these measures cannot be used to advantage.

A computer program may be stored/distributed on a suitable medium, such as an optical storage medium or a solid-state medium supplied together with or as part of other hardware, but may also be distributed in other forms, such as via the Internet or other wired or wireless telecommunication systems. (optional)

If the term "adapted to" is used in the claims or description, it is noted the term "adapted to" is intended to be equivalent to the term "configured to".

Any reference signs in the claims should not be construed as limiting the scope.

## Claims

1. A ventilation apparatus for providing breathing therapy, comprising:
a breathing gas delivery system (10,12) for delivering breathing gas to a user of the apparatus, with a controllable pressure and flow;
a pressure sensor (14) for monitoring a breathing pressure;
a flow sensor (15) for monitoring a breathing flow; and
a processor (30) adapted to:
process the monitored breathing pressure and the monitored breathing flow to derive a respiration parameter which is indicative of a relaxation state of the user;
estimate a state of relaxation of the user during delivery of breathing therapy based on the respiration parameter;
configure settings of the ventilation apparatus in dependence on the estimated state of relaxation for assisting the user in habituating to the breathing therapy;
wherein the processor is adapted to configure said settings of the ventilation apparatus to control at least an expiratory pressure level;
wherein the processor is adapted to configure the ventilation apparatus settings such as to vary the settings incrementally in steps from a sub-therapeutic level to a therapeutic level,
wherein the processor is adapted to so vary the settings incrementally to increase the pressure level from a sub-therapeutic to therapeutic level only while the user remains above a defined relaxation state threshold thₘᵢₙ;
wherein, responsive to the relaxation state falling below the relaxation state threshold thₘᵢₙ, the processor is adapted to lower the ventilation pressure in order to allow the user to return to a more relaxed state.

2. The apparatus of claim 1, wherein the respiration parameter comprises:
a respiration variability; and/or
a heart rate variability; and/or
an asynchrony index; and/or
a rapid shallow breathing index signal.

3. The apparatus of any one of claims 1 to 2, wherein the processor is adapted to further configure the settings to control one or more of:
the flow rate;
the inspiratory rise time;
the pressure rise time; and
the duration of the ventilation device pressure and/or flow periods.

4. The apparatus of any one of claims 1 to 3, wherein the processor is further adapted to provide audio and/or visual feedback to the user indicating a level of therapy habituation.

5. The apparatus of any one of claims 1 to 4, further comprising additional sensors for providing signals for use in estimating the state of relaxation of the user.

6. The apparatus of claim 5, wherein the additional sensors comprise one or more of:
a respiratory effort sensor;
a photoplethysmogram sensor for determining heart rate variability;
a galvanic skin response sensor;
a sweat or saliva sensor for measuring a Cortisol level;
a body temperature sensor;
an EMG sensor;
a respiratory effort sensor.

7. The apparatus of any one of claims 1 to 6, further comprising an output stimulation device for influencing the relaxation state of the user.

8. The apparatus of claim 7, wherein the output stimulation device comprises an audio and/or visual output device for providing one or more of:
red light stimulation;
breathing exercise guidance;
guided meditation;
mask readjustment or change advice;
auditory delivered Eye Movement Desensitization and Reprocessing (EMDR).

9. The device of any one of claims 1 to 8, wherein the processor is further adapted to provide a control signal for controlling the environmental temperature and humidity.

10. The device of any one of claims 1 to 9, wherein the processor is adapted to configure the settings during a pre-therapy training period.

11. A computer program comprising computer program code, which is adapted, when said program is run on a processor of a ventilation apparatus, to implement a control method for controlling the ventilation apparatus, wherein the control method comprises:
monitoring a breathing pressure and a breathing flow;
processing the monitored breathing pressure and the monitored breathing flow to derive a respiration parameter which is indicative of a relaxation state of the user;
estimating a state of relaxation of the user during delivery of breathing assistance based on the respiration parameter;
configuring settings of the ventilation apparatus in dependence on the estimated state of relaxation for assisting the user in habituating to the breathing therapy; and
wherein the method comprises configuring said settings to control at least an expiratory pressure level;
wherein the method comprises configuring the ventilation apparatus settings such as to vary the settings incrementally in steps from a sub-therapeutic level to a therapeutic level,
wherein the method comprises so varying the ventilation apparatus settings incrementally to increase the pressure level from a sub-therapeutic to therapeutic level only if the user remains above a defined relaxation state threshold thₘᵢₙ;
wherein, responsive to the relaxation state level falling below the relaxation state threshold thₘᵢₙ, the method comprises lowering the ventilation pressure in order to allow the user to return to a more relaxed state.

## Patentansprüche

1. Beatmungseinrichtung zum Bereitstellen einer Atemtherapie, umfassend:
ein Atemgaszufuhrsystem (10, 12) zum Zuführen von Atemgas zu einem Benutzer der Einrichtung mit einem steuerbaren Druck und Durchfluss;
einen Drucksensor (14) zum Überwachen eines Atemdrucks;
einen Durchflusssensor (15) zum Überwachen eines Atemflusses; und
einen Prozessor (30), der angepasst ist zum:
Verarbeiten des überwachten Atemdrucks und des überwachten Atemflusses, um einen Atmungsparameter abzuleiten, der einen Entspannungszustand des Benutzers angibt;
Schätzen eines Entspannungszustands des Benutzers während der Zuführung einer Atemtherapie basierend auf dem Atmungsparameter;
Konfigurieren von Einstellungen der Beatmungseinrichtung in Abhängigkeit vom geschätzten Entspannungszustand, um den Benutzer bei der Gewöhnung an die Atemtherapie zu unterstützen;
wobei der Prozessor dazu angepasst ist, die Einstellungen der Beatmungseinrichtung zum Steuern von zumindest einem Ausatmungsdruckniveau zu konfigurieren;
wobei der Prozessor dazu angepasst ist, die Einstellungen der Beatmungseinrichtung derart zu konfigurieren, dass die Einstellungen stufenweise von einem subtherapeutischen Niveau zu einem therapeutischen Niveau verändert werden,
wobei der Prozessor dazu angepasst ist, die Einstellungen stufenweise so zu verändern, dass das Druckniveau nur dann von einem subtherapeutischen auf ein therapeutisches Niveau erhöht wird, solange der Benutzer über einem definierten Entspannungszustandsschwellenwert thₘᵢₙ bleibt;
wobei der Prozessor als Reaktion darauf, dass der Entspannungszustand unter den Entspannungszustandsschwellenwert thₘᵢₙ fällt, dazu angepasst ist, den Beatmungsdruck zu senken, um dem Benutzer die Rückkehr in einen entspannteren Zustand zu ermöglichen.

2. Einrichtung nach Anspruch 1, wobei der Atmungsparameter umfasst:
eine Atmungsvariabilität; und/oder
eine Herzfrequenzvariabilität; und/oder
einen Asynchronitätsindex; und/oder
ein Signal für einen Index für schnelles, flaches Atmen.

3. Einrichtung nach einem der Ansprüche 1 bis 2, wobei der Prozessor dazu angepasst ist, die Einstellungen weiter dazu zu konfigurieren, eines oder mehrere der Folgenden zu steuern:
die Durchflussrate;
die inspiratorische Anstiegszeit;
die Druckanstiegszeit; und
die Dauer des Beatmungsvorrichtungsdrucks und/oder -durchflussperioden.

4. Einrichtung nach einem der Ansprüche 1 bis 3, wobei der Prozessor weiter dazu angepasst ist, Audiosignale und/oder visuelle Rückmeldung an den Benutzer bereitzustellen, die einen Grad der Gewöhnung an die Therapie angeben.

5. Einrichtung nach einem der Ansprüche 1 bis 4, das weiter zusätzliche Sensoren zum Bereitstellen von Signalen zur Verwendung bei der Schätzung des Entspannungszustands des Benutzers umfasst.

6. Einrichtung nach Anspruch 5, wobei die zusätzlichen Sensoren einen oder mehrere der Folgenden umfassen:
einen Atemanstrengungssensor;
einen Photoplethysmogramm-Sensor zum Bestimmen der Herzfrequenzvariabilität;
einen galvanischen Hautreaktionssensor;
einen Schweiß- oder Speichelsensor zum Messen eines Cortisolspiegels;
einen Temperatursensor;
einen EMG-Sensor;
einen Atemanstrengungssensor.

7. Einrichtung nach einem der Ansprüche 1 bis 6, die weiter eine Ausgabestimulationsvorrichtung zum Beeinflussen des Entspannungszustands des Benutzers umfasst.

8. Einrichtung nach Anspruch 7, wobei die Ausgabestimulationsvorrichtung eine Audio- und/oder visuelle Ausgabevorrichtung zum Bereitstellen eines oder mehrerer der Folgenden umfasst:
Rotlichtstimulation;
Anleitung zu Atemübungen;
geführte Meditation;
Hinweise zur Neueinstellung oder zum Wechsel der Maske;
auditiv zugeführte Desensibilisierung und Aufarbeitung durch Augenbewegungen (EMDR).

9. Vorrichtung nach einem der Ansprüche 1 bis 8, wobei der Prozessor weiter dazu angepasst ist, ein Steuersignal zum Steuern der Umgebungstemperatur und -feuchtigkeit bereitzustellen.

10. Vorrichtung nach einem der Ansprüche 1 bis 9, wobei der Prozessor dazu angepasst ist, die Einstellungen während einer Trainingsphase vor der Therapie zu konfigurieren.

11. Computerprogramm, das Computerprogrammcode umfasst, der dazu angepasst ist, wenn das Programm auf einem Prozessor einer Beatmungseinrichtung ausgeführt wird, ein Steuerverfahren zum Steuern der Beatmungseinrichtung zu implementieren, wobei das Steuerverfahren umfasst:
Überwachen eines Atemdrucks und eines Atemflusses;
Verarbeiten des überwachten Atemdrucks und des überwachten Atemflusses, um einen Atmungsparameter abzuleiten, der einen Entspannungszustand des Benutzers angibt;
Schätzen eines Entspannungszustands des Benutzers während der Zuführung einer Atemunterstützung basierend auf dem Atmungsparameter;
Konfigurieren von Einstellungen der Beatmungseinrichtung in Abhängigkeit vom geschätzten Entspannungszustand, um den Benutzer bei der Gewöhnung an die Atemtherapie zu unterstützen; und
wobei das Verfahren Konfigurieren der Einstellungen zum Steuern von zumindest einem Ausatmungsdruckniveau umfasst;
wobei das Verfahren Konfigurieren der Einstellungen der Beatmungseinrichtung derart umfasst, dass die Einstellungen stufenweise von einem subtherapeutischen Niveau zu einem therapeutischen Niveau verändert werden,
wobei das Verfahren umfasst, die Einstellungen der Beatmungseinrichtung stufenweise so zu verändern, dass das Druckniveau nur dann von einem subtherapeutischen auf ein therapeutisches Niveau erhöht wird, wenn der Benutzer über einem definierten Entspannungszustandsschwellenwert thₘᵢₙ bleibt;
wobei das Verfahren als Reaktion darauf, dass ein Entspannungszustandsniveau unter den Entspannungszustandsschwellenwert thₘᵢₙ fällt, Absenken des Beatmungsdrucks umfasst, um dem Benutzer die Rückkehr in einen entspannteren Zustand zu ermöglichen.

## Revendications

1. Appareil de ventilation pour fournir une thérapie respiratoire, comprenant :
un système de distribution de gaz respiratoire (10, 12) pour distribuer un gaz respiratoire à un utilisateur de l'appareil, avec une pression et un écoulement pouvant être régulés ;
un capteur de pression (14) pour surveiller une pression respiratoire ;
un capteur d'écoulement (15) pour surveiller un flux respiratoire ; et
un processeur (30) adapté pour :
traiter la pression respiratoire surveillée et le débit respiratoire surveillé pour déduire un paramètre de respiration qui indique un état de relaxation de l'utilisateur ;
estimer un état de relaxation de l'utilisateur pendant la délivrance d'une thérapie respiratoire sur la base du paramètre de respiration ;
configurer des réglages de l'appareil de ventilation en fonction de l'état de relaxation estimé pour aider l'utilisateur à s'habituer à la thérapie respiratoire ;
dans lequel le processeur est adapté pour configurer lesdits réglages de l'appareil de ventilation pour commander au moins un niveau de pression expiratoire ;
dans lequel le processeur est adapté pour configurer les réglages de l'appareil de ventilation de manière à faire varier les réglages de manière incrémentielle par étapes, d'un niveau sous-thérapeutique à un niveau thérapeutique,
dans lequel le processeur est adapté pour faire varier les réglages de manière incrémentielle pour augmenter le niveau de pression d'un niveau sous-thérapeutique à un niveau thérapeutique uniquement pendant que l'utilisateur reste au-dessus d'un seuil d'état de relaxation défini thₘᵢₙ ;
dans lequel, en réponse à l'état de relaxation tombant en dessous du seuil d'état de relaxation thₘᵢₙ, le processeur est adapté pour abaisser la pression de ventilation afin de permettre à l'utilisateur de revenir à un état plus relâché.

2. Appareil selon la revendication 1, dans lequel le paramètre de respiration comprend :
une variabilité de respiration ; et/ou
une variabilité de fréquence cardiaque ; et/ou
un indice d'asynchronie ; et/ou
un signal d'indice de respiration rapide et superficielle.

3. Appareil selon l'une quelconque des revendications 1 à 2, dans lequel le processeur est adapté pour configurer en outre les réglages pour commander un ou plusieurs :
du débit d'écoulement ;
du temps de montée inspiratoire ;
du temps de montée en pression ; et
de la durée de la pression de dispositif de ventilation et/ou des périodes d'écoulement.

4. Appareil selon l'une quelconque des revendications 1 à 3, dans lequel le processeur est en outre adapté pour fournir un retour audio et/ou visuel à l'utilisateur indiquant un niveau d'accoutumance à la thérapie.

5. Appareil selon l'une quelconque des revendications 1 à 4, comprenant en outre des capteurs supplémentaires pour fournir des signaux destinés à être utilisés pour estimer l'état de relaxation de l'utilisateur.

6. Appareil selon la revendication 5, dans lequel les capteurs supplémentaires comprennent un ou plusieurs :
d'un capteur d'effort respiratoire ;
d'un capteur de photopléthysmogramme pour déterminer une variabilité de fréquence cardiaque ;
d'un capteur de réponse galvanique de la peau ;
d'un capteur de sueur ou de salive pour mesurer un niveau de cortisol ;
d'un capteur de température corporelle ;
d'un capteur EMG ;
d'un capteur d'effort respiratoire.

7. Appareil selon l'une quelconque des revendications 1 à 6, comprenant en outre un dispositif de stimulation de sortie pour influencer l'état de relaxation de l'utilisateur.

8. Appareil selon la revendication 7, dans lequel le dispositif de stimulation de sortie comprend un dispositif de sortie audio et/ou visuelle pour fournir un ou plusieurs :
d'une stimulation par lumière rouge ;
de guides d'exercices de respiration ;
d'une méditation guidée ;
de conseils de réajustement ou de changement de masque ;
d'une désensibilisation et d'un retraitement par mouvements oculaires (EMDR) délivrés par voie auditive.

9. Dispositif selon l'une quelconque des revendications 1 à 8, dans lequel le processeur est en outre adapté pour fournir un signal de commande pour réguler la température et l'humidité de l'environnement.

10. Dispositif selon l'une quelconque des revendications 1 à 9, dans lequel le processeur est adapté pour configurer les réglages pendant une période de formation préthérapeutique.

11. Programme informatique comprenant un code de programme informatique qui est adapté, lorsque ledit programme est exécuté sur un processeur d'un appareil de ventilation, pour mettre en oeuvre un procédé de commande pour commander l'appareil de ventilation, dans lequel le procédé de commande comprend :
la surveillance d'une pression respiratoire et d'un flux respiratoire ;
le traitement de la pression respiratoire surveillée et du flux respiratoire surveillés pour déduire un paramètre de respiration qui indique un état de relaxation de l'utilisateur ;
l'estimation d'un état de relaxation de l'utilisateur pendant la délivrance d'une assistance respiratoire sur la base du paramètre de respiration ;
la configuration de réglages de l'appareil de ventilation en fonction de l'état de relaxation estimé pour aider l'utilisateur à s'habituer à la thérapie respiratoire ; et
dans lequel le procédé comprend la configuration desdits réglages pour commander au moins un niveau de pression expiratoire ;
dans lequel le procédé comprend la configuration des réglages de l'appareil de ventilation de manière à faire varier les réglages de manière incrémentielle par étapes depuis un niveau sous-thérapeutique jusqu'à un niveau thérapeutique,
dans lequel le procédé comprend la variation des réglages de l'appareil de ventilation de manière incrémentielle pour augmenter le niveau de pression d'un niveau sous-thérapeutique à un niveau thérapeutique uniquement si l'utilisateur reste au-dessus d'un seuil d'état de relaxation défini thₘᵢₙ ;
dans lequel, en réponse au niveau d'état de relaxation tombant en dessous du seuil d'état de relaxation thₘᵢₙ, le procédé comprend l'abaissement de la pression de ventilation afin de permettre à l'utilisateur de revenir à un état plus relâché.
